# EUROPÄISCHE PATENTSCHRIFT

(11) **EP 0 612 747 B1**
(45) Veröffentlichungstag und Bekanntmachung des Hinweises auf die Patenterteilung: **07.01.1998**
(21) Anmeldenummer: 94810087.0
(22) Anmeldetag: 15.02.1994
(51) Int. Cl.: C07D 487/04, C07D 519/00, C09B 57/00, C08K 5/3415

(54) **Nitroxylgruppen enthaltende Diketopyrrolopyrrole**
Nitroxyl groups-containing diketopyrrolopyrroles
Dicétopyrrolopyrroles contenant des groupes nitroxyles

(30) Priorität: 23.02.1993 CH 552/93
(43) Veröffentlichungstag der Anmeldung: 31.08.1994
(73) Patentinhaber: Ciba Specialty Chemicals Holding Inc., 4057 Basel (CH)
(72) Erfinder: Chassot, Laurent, Dr., CH-1724 Praroman (CH); Wooden, Gary, Dr., CH-1716 Oberschrot (CH)

(56) Entgegenhaltungen:
- WO-A-92/12201
- US-A- 3 970 632
- US-A- 4 415 685

## Beschreibung

Die vorliegende Erfindung betrifft Nitroxyl- beziehungsweise N-hydrocarbyloxyaminogruppen enthaltende Diketopyrrolopyrrole und ihre Verwendung als Pigmente oder als Lichtschutzmittel für andere Diketopyrrolopyrrole.

Pyrrolo-[3,4-c]-pyrrolpigmente sind seit einigen Jahren bekannt und werden z.B. in US-Patent 4 415 685 und US-Patent 4 579 949 beschrieben. Einige davon haben sich als hochwertige Pigmente bewährt. Obwohl sie bereits eine sehr gute Licht- und Wetterbeständigkeit zeigen, ist nun gefunden worden, dass diese überraschenderweise ohne Beeinträchtigung anderer Eigenschaften noch verbessert werden können, insbesondere bei transparenten Pigmentformen, wenn in das Diketopyrrolopyrrolmolekül eine Nitroxyl- oder eine N-hydrocarbyloxyaminogruppe eingeführt wird oder wenn einem üblichen Diketopyrrolopyrrol ein derartig modifiziertes Produkt beigemischt wird.

Nitroxylverbindungen sind aus verschiedenen Publikationen als Stabilisatoren bekannt. In JP-A 75-58141 werden unter vielen anderen HALS (Hindered Amine Light Stabiliser) auch Nitroxylverbindungen beschrieben, die zusammen mit UV-Absorbern zur Stabilisierung von pigmentierten Kunststoffen gegen den Einfluss von Licht dienen. Es wird ausgeführt, dass die Wirkung der UV-Absorber als Lichtstabilisatoren für den Kunststoff durch die Pigmente beeinträchtigt wird, dass aber diese Beeinträchtigung durch einen HALS vermindert werden kann. Bevorzugt werden HALS, die keine Nitroxylgruppe enthalten. In US-Patent 3 970 632 wird eine Polymermasse enthaltend ein Chinophthalonpigment, welche dank Zugabe von HALS eine hohe Lichtbeständigkeit und Verblassungsfestigkeit aufweist, beschrieben. Obwohl ganz generisch auch Nitroxylverbindungen mit umfasst werden, sind spezifisch nur nitroxylgruppenfreie HALS aufgeführt. Aus JP-A 82-119941 sind pigmentierte Polymere enthaltend einen UV-Absorber und einen HALS (ohne Hinweis auf Nitroxylverbindungen) zur Vorbeugung von Verblassung bekannt. In JP-A 90-99323 werden gegen die Verblassung durch die Einwirkung von Licht stabilisierte Laminate beschrieben, die in einer Zwischenschicht einen in einem organischen Lösungsmittel löslichen Farbstoff (irrtümlicherweise als Pigment bezeichnet) enthalten, der in gelöster Form mit einer Nitroxylverbindung behandelt wurde. Aus Dyes and Pigments 17 (1991), 83-100, sind Nitroxyl-HALS-Gruppen enthaltende Farbstoffe und aus US-Patent 4 866 113 Nitroxylfreie-HALS-Gruppen enthaltende organische Pigmente bekannt.

EP-A 309 401 beschreibt die Verwendung von N-Hydroxy-HALS zur Vermeidung von Versprödung, Sprungbildung, Korrosion, Erosion, Glanzverlust, Auskreiden und Vergilbung in bestimmte organische Pigmente, wie Phthalocyanin- und Azopigmente, enthaltenden Beschichtungen.

N-hydrocarbyloxy-Derivate von HALS sind z.B. in den US Patenten 4 921 962, 5 019 613, 5 021 483, 5 021 486, 5 021 577 und in EP-A 389 428 als Lichtstabilisatoren für Polymere offenbart. Es wird dabei ausschliesslich auf deren Schutzeffekt gegen die zersetzende Wirkung des Lichtes auf hochmolekularem organischem Material, wie Polyolefine, Elastomere, Polyvinylchlorid, Polyester, Polyurethane, bei Raumtemperatur und säurekatalytisch thermisch härtbare Beschichtungen und Emails, hingewiesen.

Aus diesen Publikationen ergibt sich für den Fachmann die Lehre, dass bei der Verwendung von löslichen Farbkörpern, die selbst eine Nitroxylgruppe enthalten oder denen eine nitroxylgruppenhaltige Verbindung beigemengt wird, die Verblassung durch Einwirkung von Licht vermindert werden kann, dass der gleiche Effekt aber im Falle der Pigmente, wo auch andere Eigenschaften, wie z.B. Dispergierbarkeit, Hitze- und Migrationsbeständigkeit, eine wesentliche Rolle spielen, durch die Anwesenheit nitroxylgruppenfreier HALS erzielt wird.

Es sind nun neue Nitroxyl- beziehungsweise N-hydrocarbyloxyaminogruppen enthaltende Diketopyrrolopyrrole gefunden worden, die im Vergleich zu herkömmlichen Diketopyrrolopyrrolen überraschenderweise ohne Beeinträchtigung anderer unerlässlicher Pigmenteigenschaften, eine noch verbesserte Licht- und Wetterbeständigkeit zeigen.

Die vorliegende Erfindung betrifft demnach Diketopyrrolopyrrole der Formel 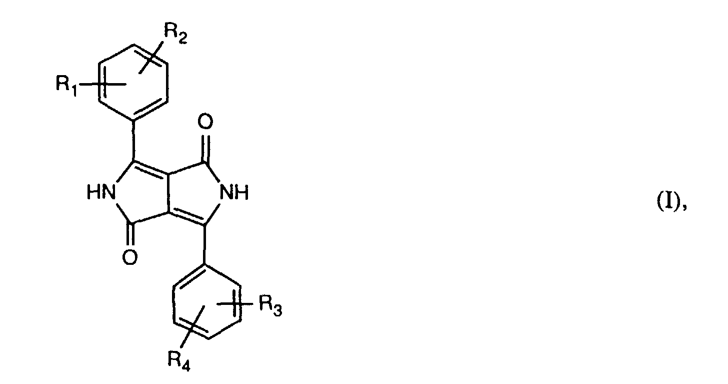 worin
R₁, R₂, R₃ und R₄ unabhängig voneinander Wasserstoff, Chlor, C₁-C₄-Alkyl, Methoxy, Phenyl, Cyano oder eine Gruppe

-X₁-V-X₂-T-A

bedeuten, wobei mindestens einer von R₁, R₂, R₃ oder R₄ die Gruppe A enthält,
X₁ und X₂ unabhängig voneinander -O-, -S-, -N(R₅)-, -CO- oder -SO₂- oder eine direkte Bindung,
V ein Gruppe -(CH₂)ₘ-, 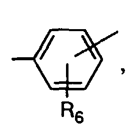 -(CH₂CH₂O)ₘ-CH₂CH₂- oder eine direkte Bindung und m 1 oder 2 bedeuten, wobei, wenn V eine direkte Bindung ist, X₁ ebenfalls eine direkte Bindung bedeutet,
T eine Gruppe 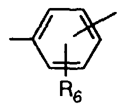 oder -(CH₂)ₘ- ist und, wenn nicht an einem N-Atom gebunden, auch eine direkte Bindung sein kann,
R₅ Wasserstoff oder C₁-C₄-Alkyl und
R₆ Waserstoff, Halogen oder C₁-C₄-Alkyl bedeuten,
A eine Gruppe der Formel 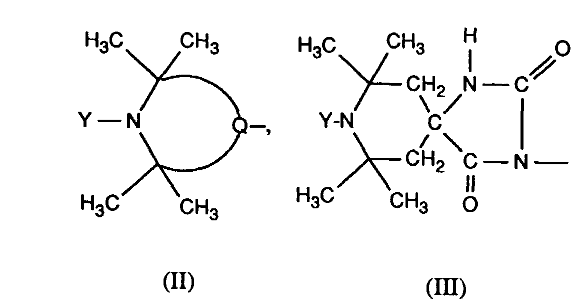 oder 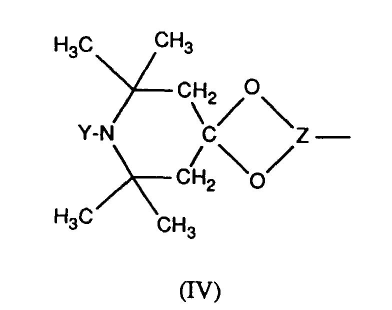 ist, worin
Y O·, OH oder OR ist und R C₁-C₁₂-Alkyl oder C₅-C₁₂-Cycloalkyl bedeutet,
Q eine Gruppe der Formel 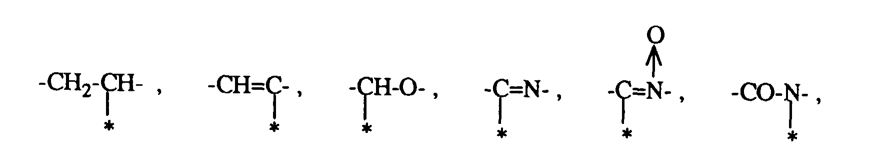 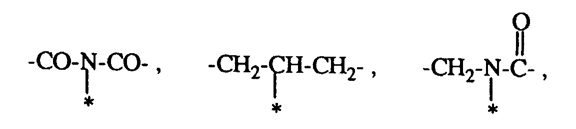 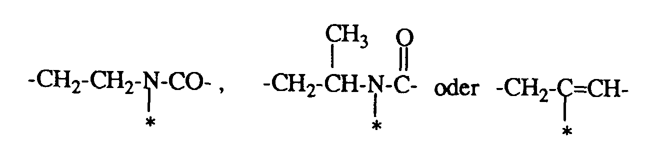 darstellt, worin die mit * bezeichnete Bindung die zu T führende Bindung bedeutet, und
Z eine Gruppe der Formel 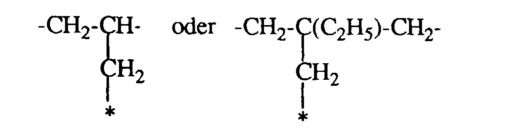 ist, worin die mit * bezeichnete Bindung die zu T führende Bindung darstellt.

Bedeuten etwaige Substituenten C₁-C₄-Alkyl, so handelt es sich um Methyl, Ethyl, n-Propyl, Isopropyl, n-Butyl, sec.-Butyl oder tert.-Butyl.

Als Halogen bedeutet R₇ z.B. Brom oder insbesondere Chlor.

R bedeutet als C₅-C₁₂-Cycloalkyl z.B. Cyclopentyl, Cycloheptyl, Cyclodecyl oder insbesondere Cyclohexyl und als C₁-C₁₂-Alkyl z.B. Methyl, Ethyl, n-Propyl, Isopropyl, n-Butyl, se.-Butyl, tert.-Butyl, n-Amyl, tert.-Amyl, n-Hexyl, n-Heptyl, n-Octyl, n-Decyl oder n-Dodecyl.

Y ist vorzugsweise OR, worin R tert.-Butyl, C₇-C₁₂-Alkyl oder C₅-C₆-Cycloalkyl bedeutet, oder insbesondere O·.

Bevorzugt sind unsymmetrische Verbindungen, der Formel I, worin nur einer der Reste R₃ oder R₄ eine Gruppe

-X₁-V-X₂-T-A

bedeutet.

Von besonderem Interesse sind Diketopyrrolopyrrole der Formel 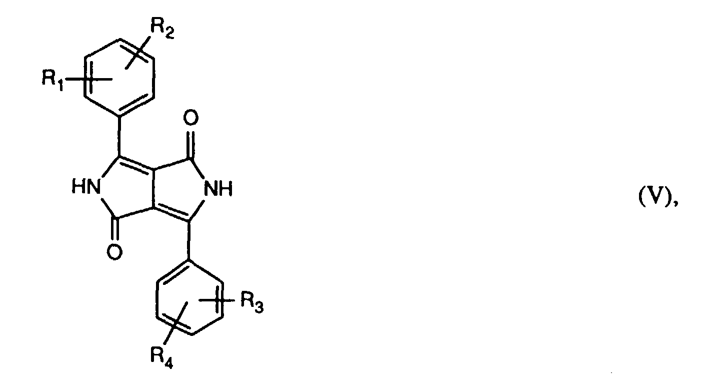 worin R₃ eine Gruppe

-X₂-T-A

bedeutet,
R₁, R₂ und R₄ unabhängig voneinander Wasserstoff, Chlor, Methyl, Methoxy, Cyano oder Phenyl sind und R₁ zusätzlich auch eine Gruppe

-X₂-T-A

bedeuten kann,
X₂ -O- oder -N(R₅)- ist,
T eine Gruppe 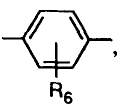 -(CH₂)ₘ- oder eine direkte Bindung bedeutet,
R₅ und R₆ Wasserstoff oder Methyl sind,
A eine Gruppe 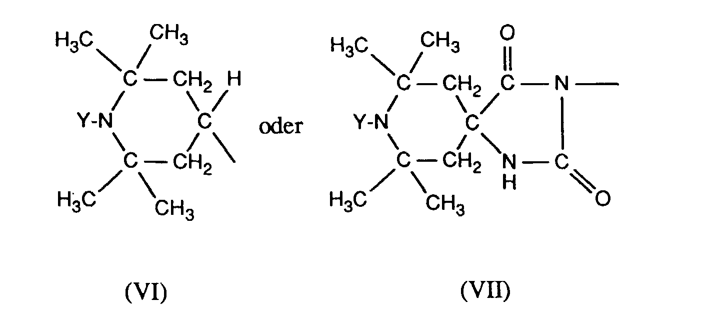 bedeutet, worin
Y O· oder OR ist und R tert.-Butyl, n-Octyl oder Cyclohexyl bedeutet.

Bevorzugt werden Diketopyrrolopyrrole der Formel 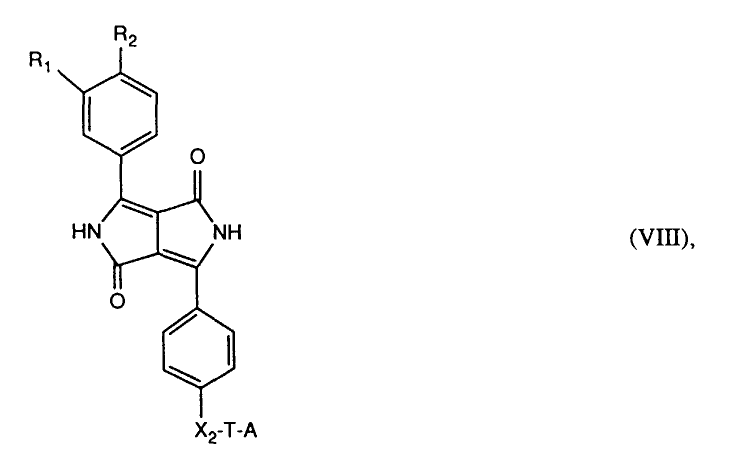 worin einer von R₁ und R₂ Wasserstoff, Chlor, Methyl, Phenyl oder Cyano und der andere Wasserstoff bedeutet,
X₂ -O- bedeutet
T eine Gruppe 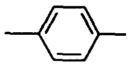 oder eine direkte Bindung ist und
A eine Gruppe der Formel VI bedeutet, worin Y O· ist.

Die Diketopyrrolopyrrole der Formel I können in Analogie zu allgemein bekannten Verfahren hergestellt werden, z.B. durch Umsetzung eines Bernsteinsäurediesters mit einem Nitril oder einem Gemisch von Nitrilen der Formeln 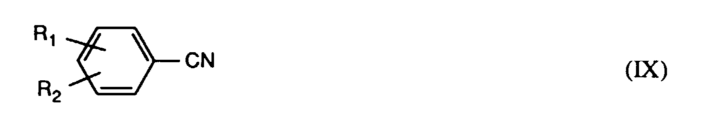 und 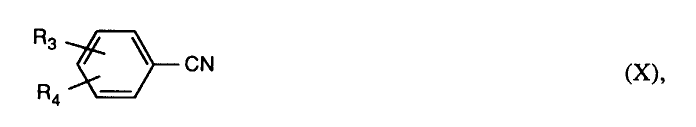 wie beispielsweise aus den US-Patenten 4 579 949 und 4 720 305 bekannt, oder insbesondere im Falle der bevorzugten Verbindungen der Formel I, worin nur einer der Reste R₃ oder R₄ eine Gruppe

-X₁-V-X₂-T-A

bedeutet, durch Umsetzung eines Pyrrolinons der Formel 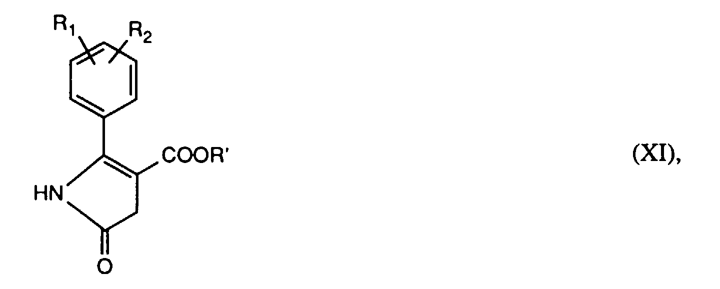 oder eines Enamins der Formel 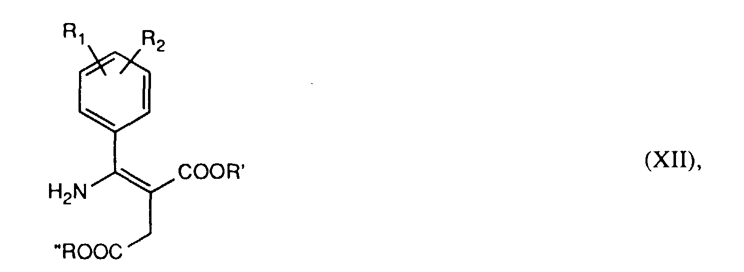 mit einem Nitril der Formel X, wie z.B. aus den US-Patenten 4 659 775 und 4 749 795 bekannt ist.

R₁, R₂, R₃ und R₄ haben in den Formeln IX, X, XI und XII die oben angegebene Bedeutung, wobei mindestens einer dieser Reste und bevorzugt einer von R₃ und R₄ eine Gruppe

-X₁-V-X₂-T-A

mit der oben angegebenen Bedeutung sein muss.

R' und R'' in den Formeln XI und XII bedeuten unabhängig voneinander Niederalkyl, z.B. Methyl, Ethyl, n-Propyl, Isopropyl, n-Butyl, sek.-Butyl, tert.-Butyl und tert.-Amyl. Bevorzugt sind Methyl und Ethyl.

Bei den Verbindungen der Formeln IX, X, XI und XII handelt es sich um bekannte Verbindungen. Sollten einige davon noch neu sein, so können sie in Analogie zu allgemein bekannten Methoden hergestellt werden. Auch Nitrile der Formeln IX und X, worin mindestens einer von R₁ und R₂ oder von R₃ und R₄ eine Gruppe -Y₁-V-X₂-T-A bedeutet, sind entweder bekannt oder können nach allgemein bekannten Methoden hergestellt werden, z.B. durch Umsetzung eines Nitrils der Formel IX oder X, worin mindestens einer von R₁ und R₂ oder R₃ und R₄ -X₁-V-X₂H bedeutet, mit einer an sich bekannten Verbindung der Formel

Cl-T-A

Zur Herstellung von Nitrilen mit -X₁-V-X₂-T-A in Parastellung kann man auch Parachlorbenzonitril mit einer Verbindung der Formel

H-X₁-V-X₂-T-A

umsetzen.

Die Diketopyrrolopyrrole der Formel I eignen sich ausgezeichnet als Pigmente zum Färben von hochmolekularem organischem Material. Sie können aber auch als Stabilisatoren von herkömmlichen Diketopyrrolopyrrolpigmenten gegen die Einwirkung von Licht und Wetter, die sowohl zur Verblassung, gegebenenfalls aber auch zum Nachdunkeln der Ausfärbungen führen kann, eingesetzt werden.

Ein weiterer Gegenstand der Erfindung sind demnach Pigmentzusammensetzungen enthaltend
a) mindestens ein Diketopyrrolopyrrol der Formel 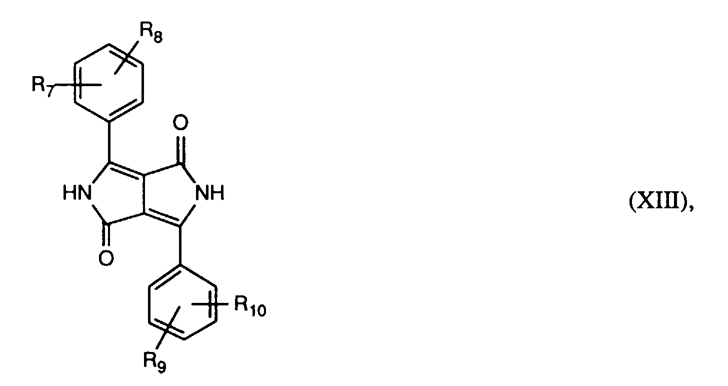 worin R₇, R₈, R₉ und R₁₀ unabhängig voneinander Wasserstoff, Chlor, C₁-C₄-Alkyl, Methoxy, Phenyl oder Cyano bedeuten und
b) 0,1 bis 20 Gew.%, bezogen auf das Diketopyrrolopyrrol a) eines Diketopyrrolopyrrols der Formel I.

Die erfindungsgemässen Pigmentzusammensetzungen erhält man durch Vermischen der beiden Komponenten a) und b).

Das Vermischen der beiden Komponenten a) und b) erfolgt nach beliebigen, allgemein bekannten Methoden. Die Komponente b) kann z.B. als feuchter Presskuchen oder als Pulver während der Synthese, der Rektristallisation oder der Filtration der Komponente a) dieser letzteren beigemischt werden. Die Komponenten a) und b) können auch durch intensives Mischen bzw. Mahlen vermischt werden oder sie können getrennt dem zu färbenden hochmolekularen organischen Material zugegeben werden und während des Dispersionsprozesses vermischt werden.

Die Diketopyrrolopyrrole gemäss der Komponente a) der erfindungsgemässen Pigmentzusammensetzungen stellen bekannte Produkte dar und können beispielsweise gemäss den in den US-Patenten 4 579 949 und 4 749 795 beschriebenen Verfahren hergestellt werden.

Die erfindungsgemässen Pigmentzusammensetzungen eignen sich wie die erfindungsgemässen Diketopyrrolopyrrole ausgezeichnet als Pigmente zum Färben von hochmolekularem organischem Material, insbesondere, wenn hohe Anforderungen an die Licht- und Wetterbeständigkeit gestellt werden.

Hochmolekulare organiche Materialien, die mit den erfindungsgemässen Pigmentzusammensetzungen pigmentiert werden können, sind z.B. Celluloseether und -ester, wie Ethylcellulose, Nitrocellulose, Celluloseacetat, Cellulosebutyrat, natürliche Harze oder Kunstharze, wie Polymerisationsharze oder Kondensationsharze, wie Aminoplaste, insbesondere Harnstoff- und Melamin-Formaldehydharze, Alkydharze, Phenoplaste, Polycarbonate, Polyolefine, Polystyrol, Polyvinylchlorid, Polyamide, Polyurethane, Polyester, ABS, Polyphenylenoxide, Gummi, Casein, Silikon und Silikonharze, einzeln oder in Mischungen.

Besonders geeignet sind die erfindungsgemässen Diketopyrrolopyrrole und Pigmentzusammensetzungen zum Einfärben von Polyvinylchlorid und Polyolefinen, wie Polyethylen und Polypropylen, sowie zum Pigmentieren von Lacken, Anstrichstoffen und Druckfarben. Dank ihrer hohen Lichtbeständigkeit eignen sie sich aber auch sehr gut für den Einsatz in elektrophotographischen Materialien (z.B. Photozellen), Farbfiltern (z.B. Flüssigkristallanzeigen), Informationsspeicherungsmaterialien ("optical disc"), nicht linearer Optik und in optischen Begrenzern ("optical limiters").

Die erfindungsgemässen Diketopyrrolopyrrole und Pigmentzusammensetzungen eignen sich aber vorzugsweise zum Färben von wässrigen und/oder lösungsmittelhaltigen Lacken, insbesondere Automobillacken. Ganz besonders bevorzugt ist ihre Verwendung für Effektlackierungen, bei denen transparente organische Pigmente eingesetzt werden.

Die erwähnten hochmolekularen organischen Verbindungen können einzeln oder in Gemischen als plastische Massen, Schmelzen oder in Form von Spinnlösungen, Lacken, Anstrichstoffen oder Druckfarben vorliegen. Je nach Verwendungszweck erweist es sich als vorteilhaft, die erfindungsgemässen Diketopyrrolopyrrole oder Pigmentzusammensetzungen als Toner oder in Form von Präparaten einzusetzen.

Bezogen auf das zu pigmentierende hochmolekulare organische Material kann man die erfindungsgemässen Diketopyrrolopyrrole oder Pigmentzusammmensetzungen in einer Menge von 0,01 bis 40 Gew.%, vorzugsweise von 0,1 bis 20 Gew.%, einsetzen.

Die Pigmentierung der hochmolekularen organischen Substanzen mit den erfindungsgemässen Diketopyrrolopyrrole oder Pigmentzusammensetzungen erfolgt beispielsweise derart, dass man ein solches Diketopyrrolopyrrol oder eine solche Pigmentzusammensetzung gegebenenfalls in Form von Masterbatches, oder die einzelnen Komponenten diesen Substraten unter Verwendung von Walzwerken, Misch- oder Mahlapparaten zumischt. Das pigmentierte Material wird hierauf nach an sich bekannten Verfahren, wie Kalandrieren, Pressen, Strangpressen, Streichen, Giessen oder Spritzgiessen, in die gewünschte endgültige Form gebracht. Oft ist es erwünscht, zur Herstellung von nicht starren Formlingen oder zur Verringerung ihrer Sprödigkeit den hochmolekularen Verbindungen vor der Verformung sogenannte Weichmacher einzuverleiben. Als solche können z.B. Ester der Phosphorsäure, Phthalsäure oder Sebacinsäure dienen. Die Weichmacher können vor oder nach der Einverleibung der erfindungsgemässen Diketopyrrolopyrrole oder Pigmentzusammensetzungen in die Polymeren eingearbeitet werden. Zwecks Erzielung verschiedener Farbtöne ist es ferner möglich, den hochmolekularen organischen Stoffen neben den erfindungsgemässen Diketopyrrolopyrrole oder Pigmentzusammensetzungen noch Füllstoffe beziehungsweise andere farbgebende Bestandteile, wie Weiss-, Bunt- oder Schwarzpigmente, in beliebigen Mengen zuzufügen.

Zum Pigmentieren von Lacken, Anstrichstoffen und Druckfarben werden die hochmolekularen organischen Materialien und die erfindungsgemässen Diketopyrrolopyrrole oder Pigmentzusammensetzungen gegebenenfalls zusammen mit Zusatzstoffen, wie Füllmitteln, anderen Pigmenten, Siccativen oder Weichmachern, in einem gemeinsamen organischen Lösungsmittel oder Lösungsmittelgemisch fein dispergiert beziehungsweise gelöst. Man kann dabei so verfahren, dass man die einzelnen Komponenten für sich oder auch mehrere gemeinsam dispergiert beziehungsweise löst, und erst hierauf alle Komponenten zusammenbringt.

In Färbungen, beispielsweise von Lacken, Polyvinylchlorid oder Polyolefinen, zeichnen sich die erfindungsgemässen Diketopyrrolopyrrole sowie die erfindungsgemässen Pigmentzusammensetzungen durch gute allgemeine Pigmenteigenschaften, wie gute Dispergierbarkeit, hohe Farbstärke und Reinheit, gute Migrations-, Hitze- und insbesondere Licht- und Wetterbeständigkeit aus.

Die nachfolgenden Beispiele erläutern die Erfindung. Darin bedeuten Teile Gewichtsteile, sofern nichts anderes angegeben ist.

Beispiel 1a): Eine Lösung von 20,6 g 4-Chlorbenzonitril und 25,8 g eines Alkohols der Formel 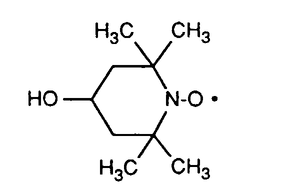 in 50 ml N-Methylpyrrolidon wird unter Rühren einer Suspension von 4,3 g Natriumhydrid in 20 ml N-Methylpyrrolidon innerhalb von 10 Minuten zugegeben. Die erhaltene Suspension wird 16 Stunden bei 95°C weitergerührt. Dann werden 300 ml Essigsäureethylester zugegeben und die organische Phase wird mit Wasser (3x100 ml) extrahiert und über Na₂SO₄ getrocknet. Nach Abdestillieren des Essigsäureethylesters und Trocknen im Vakuum erhält man 18,3 g des Nitrils der Formel 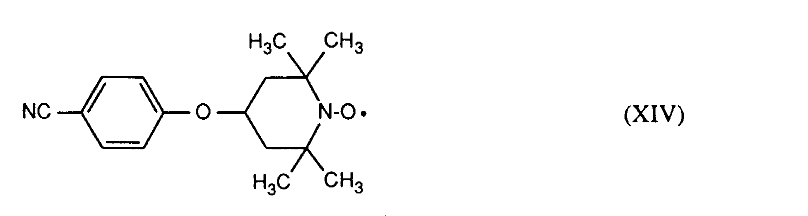 mit Schmelzpunkt 141°C

| Analyse | C | H | N |
|---|---|---|---|
| Berechnet | 70,30 % | 7,74 % | 10,25 % |
| Gefunden | 70,35 % | 7,85 % | 10,12 % |

b): 2,75 g Natrium werden unter kräftigem Rühren in 96 ml tert.-Amylalkohol bis zur vollständigen Umsetzung unter Rückfluss erhitzt, dann werden zuerst 8,2 g des gemäss a) erhaltenen Nitrils der Formel XIV und danach 8,28 g des Pyrrolinons der Formel 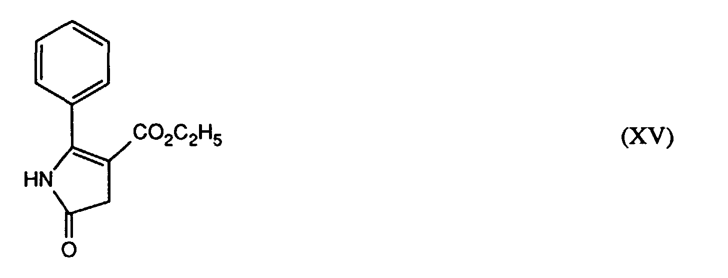 in 4 Portionen innerhalb einer Stunde stets unter Rückfluss der Lösung zugegeben. Die entstehende dunkelrote Mischung wird 2 weitere Stunden am Rückfluss erhitzt, dann auf Raumtemperatur abgekühlt und mit 100 ml Wasser verdünnt. Danach wird der pH mit Essigsäure auf 7 gestellt. Die Reaktionsmischung wird filtriert und der Rückstand wird zuerst mit Methanol, dann mit Wasser gewaschen und im Vakuumtrockenschrank über Nacht bei 90°C getrocknet. Man erhält 3,4 g eines orangeroten Pigments der Formel

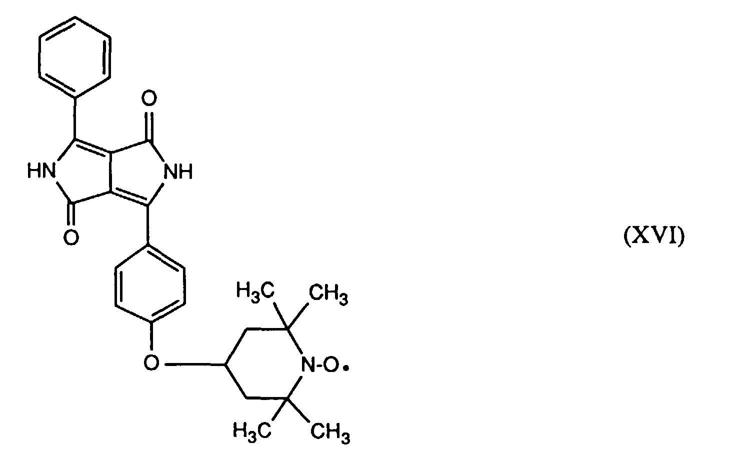

| Analyse | C | H | N |
|---|---|---|---|
| Berechnet | 70,72 | 6,16 | 9,16 |
| Gefunden | 70,16 | 6,50 | 8,68 |

Beispiel 2: Beispiel 1 wird wiederholt mit der einzigen Ausnahme, dass das Nitril der Formel XIV mit einer äquivalenten Menge eines Nitrils der Formel 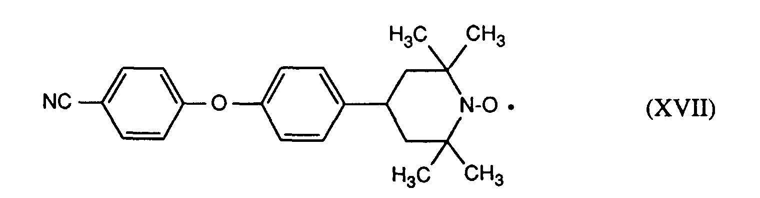 ersetzt wird. Man erhält ein Pigment der Formel

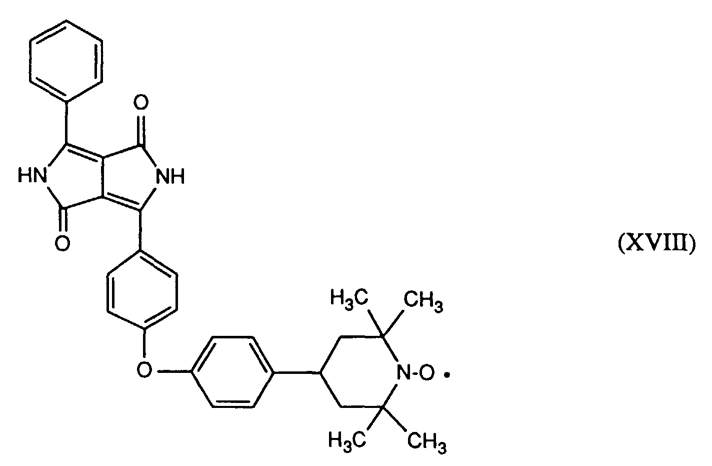

Beispiel 3: 45,65 g eines feuchten Presskuchens mit 21,9 % Festgehalt des Diketopyrrolopyrrolpigments der Formel 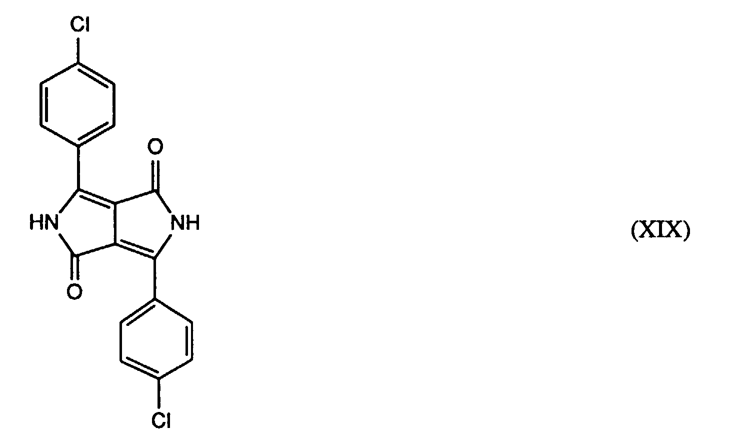 werden durch kräftiges Rühren während 30 Minuten in 200 ml Wasser suspendiert. Dann wird innerhalb von 20 Minuten eine Lösung von 0,5 g des Produktes von Beispiel 1 in 50 ml N-Methylpyrrolidon der Suspension zugetropft. Die erhaltene Suspension wird 1 Stunde auf 50°C erwärmt, dann auf Zimmertemperatur abgekühlt, filtriert, mit Wasser gewaschen und im Vakuumtrockenschrank bei 80°C getrocknet. Man erhält 11,56 g eines roten Pigmentgemisches.

Beispiel 4: Beispiel 3 wird wiederholt indem anstelle des dort eingesetzten Diketopyrrolopyrrols der Formel XIX die gleiche Gewichtsmenge des Diketopyrrolopyrrols der Formel 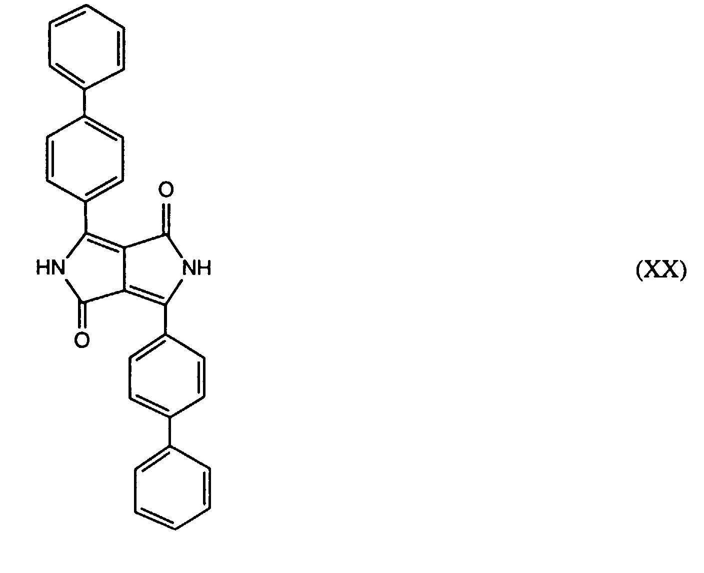 mit 2 Gew.% (bezogen auf XX) des Produktes von Beispiel 1 eingesetzt werden.

Beispiel 5: Beispiel 4 wird wiederholt, mit der einzigen Ausnahme, dass anstelle des Produktes von Beispiel 1 die gleiche Gewichtsmenge des Produktes von Beispiel 2 eingesetzt wird.

Beispiel 6: Das gemäss Beispiel 1 erhaltene Pigment wird wie folgt in einen Alkyd-Melamin-Einbrennlack eingearbeitet:

0,4 g Pigment, 7,6 g TiO₂, 9 ml Methylisobutylketon und 30 g Einbrennlack bestehend aus 66,5 Teilen Alkydharz® ALKYDAL F27 (Bayer AG), 24,4 Teilen Melaminharz ®MAPRENAL TTK (Hoechst AG), 2,1 Teilen Xylol, 4,0 Teilen Ethylenglykol und 1,0 Teil Silikonöl (1 %ig in Xylol) werden nach üblichen Methoden vermischt. Der erhaltene Farblack wird auf Aluminiumblech ausgezogen und 30 Minuten bei 130°C eingebrannt.

Weitere Lackausfärbungen werden in der genau gleichen Weise hergestellt, mit der einzigen Ausnahme, dass anstelle des Produktes von Beispiel 1 jeweils die gleiche Gewichtsmenge der Produkte der Beispiele 2, 3, 4 und 5 eingesetzt wird.

Kontroll-Lackausfärbungen werden ebenfalls in der gleichen Weise hergestellt, mit der Ausnahme, dass herkömmliche Pigmente der Formeln 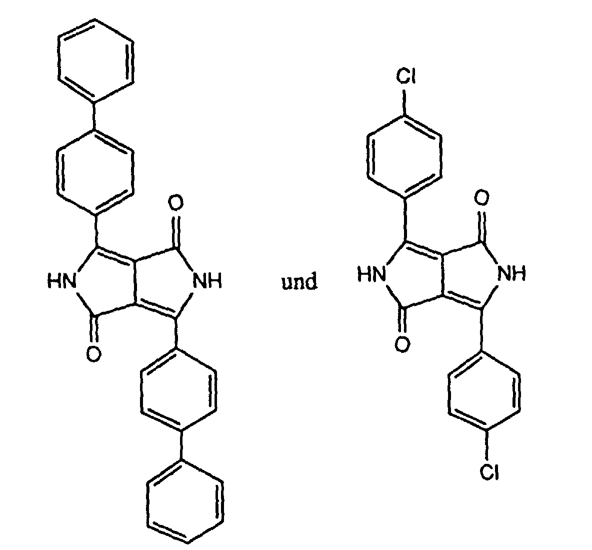 anstelle der modifizierten erfindungsgemässen Pigmente eingesetzt werden.

Die Wetterbeständigkeit der erhaltenen Lackausfärbungen wird gemäss WOM-Test nach DIN 53387 nach 500 Stunden Bewitterung bestimmt. Bei allen aufgeführten Beispielen kann eine bessere Wetterbeständigkeit, als bei den Kontroll-Lackausfärbungen nachgewiesen werden.

Beispiel 7: 22 g des Diketopyrrolopyrrolpigments der Formel 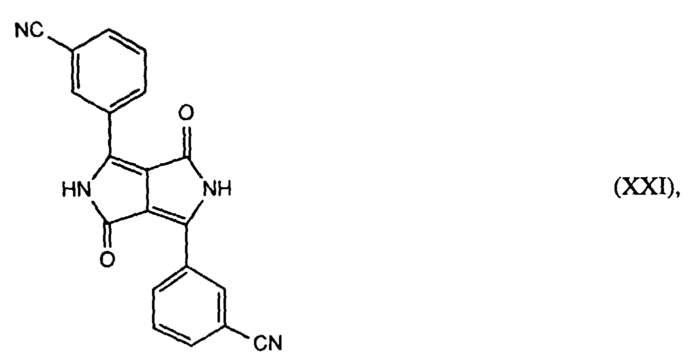 1,1 g des Pigments der Formel XVI (Beispiel 1) und 88 g NaCl werden in einem Laborkneter vorgelegt, dann werden 25 ml N-Methylpyrrolidon zugegeben und das Gemisch wird 6 Stunden bei 65°C geknetet. Danach wird die Masse auf ca. 1 1 Wasser ausgetragen und mit Zahnscheibenrührer während 3 Stunden zu einer feinen Suspension verrührt. Dann wird abfiltriert und mit Wasser salzfrei gewaschen und im Vakuumtrockenschrank getrocknet.

### Beispiel 8:

### Lösung A

| Formulierung: | |
|---|---|
| 67,1 Teile A1 | 8,2 %ige Lösung von 86 Teilen Celluloseacetobutyrat (25%ig in Butylacetat), 4 Teilen Zirkoniumoctoat, 48 Teilen SOLVESSO 150® (aromatisches Lösungsmittel von ESSO), 70 Teilen Butylacetat und 52 Teilen Xylol; |
| 24,8 Teile A2 | Polyesterharz DYNAPOL H 700® (60%ig, von Dynamit Nobel); |
| 3,1 Teile A3 | Melaminharz MAPRENAL MF 650® (55%ig, von Hoechst AG). |

### Dispersion B

| Formulierung: | |
|---|---|
| 12,3 Teile | Alu-Paste Silverline 3334® (Silverline); |
| 8,0 Teile | SOLVESSO 150®; |
| 59,34 Teile | A1; |
| 21,92 Teile | A2; |
| 2,74 Teile | A3. |

Eine 1:1 Mischung aus a) einer nach üblichen Methoden erhaltenen Dispersion von 10 Teilen der Pigmentmischung von Beispiel 7 in Lösung A und b) der Dispersion B wird mit der Spritzpistole appliziert.
Nach kurzem Ablüften wird ein Klarlack auf Basis eines hitzehärtbaren Acryllacks appliziert und bei 130°C während 30 Minuten eingebrannt. Man erhält eine orangerote Metalleffektlackierung.

Eine Kontroll-Lackausfärbung wird in der gleichen Weise hergestellt, mit der Ausnahme, dass 10 Teile des Pigments der Formel XXI ohne Zusatz von Pigment der Formel XVI eingesetzt wird.

Die Wetterbeständigkeit der erhaltenen Lackausfärbungen wird gemäss WOM-Test nach 2000 Stunden Bewitterung bestimmt.

Die Lackausfärbung enthaltend das Pigment der Formel XVI ergibt eine bessere Wetterbeständigkeit, als diejenige der Kontroll-Lackausfärbung.

## Patentansprüche

1. Diketopyrrolopyrrole der Formel 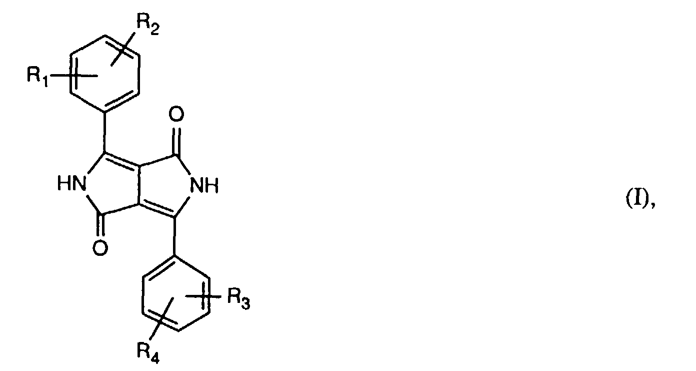 worin
R₁, R₂, R₃ und R₄ unabhängig voneinander Wasserstoff, Chlor, C₁-C₄-Alkyl, Methoxy, Phenyl, Cyano oder eine Gruppe
-X₁-V-X₂-T-A
bedeuten, wobei mindestens einer von R₁, R₂, R₃ oder R₄ die Gruppe A enthält,
X₁ und X₂ unabhängig voneinander -O-, -S-, -N(R₅)-, -CO- oder -SO₂- oder eine direkte Bindung,
V eine Gruppe -(CH₂)ₘ-, 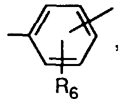 -(CH₂CH₂O)ₘ-CH₂CH₂- oder eine direkte
Bindung und m 1 oder 2 bedeuten, wobei, wenn V eine direkte Bindung ist, X₁ ebenfalls eine direkte Bindung bedeutet,
T ein Gruppe 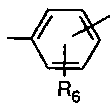 oder -(CH₂)ₘ- ist und, wenn nicht an einem N-Atom gebunden, auch eine direkte Bindung sein kann,
R₅ Wasserstoff oder C₁-C₄-Alkyl und
R₆ Waserstoff, Halogen oder C₁-C₄-Alkyl bedeuten,
A eine Gruppe der Formel 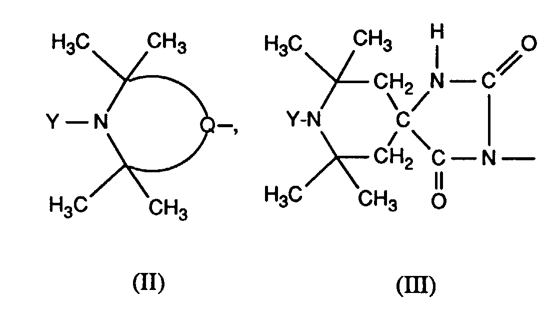 oder 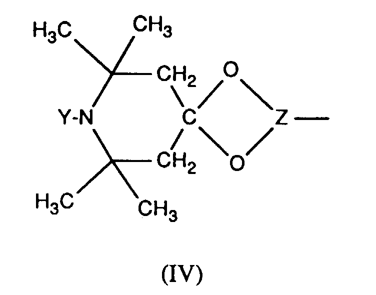 ist, worin
Y O·, OH oder OR ist und R C₁-C₁₂-Alkyl oder C₅-C₁₂-Cycioallryl bedeutet,
Q eine Gruppe der Formel 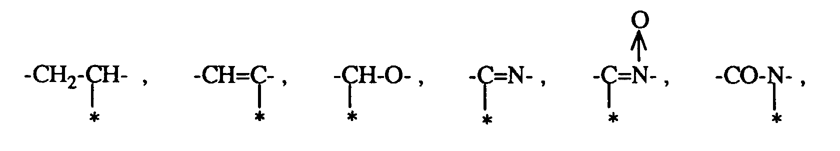 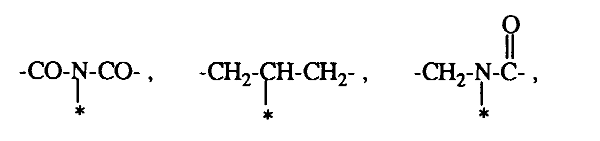 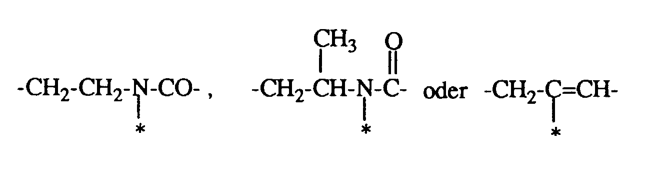 darstellt, worin die mit * bezeichnete Bindung die zu T führende Bindung bedeutet, und
Z eine Gruppe der Formel 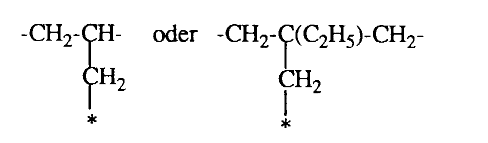 ist, worin die mit * bezeichnete Bindung die zu T führende Bindung darstellt.

2. Diketopyrrolopyrrole gemäss Anspruch 1, der Formel I, worin
Y O· oder OR und R tert.-Butyl, C₇-C₁₂-Alkyl oder C₅-C₆-Cycloalkyl bedeuten.

3. Diketopyrrolopyrrole gemäss Anspruch 2, der Formel 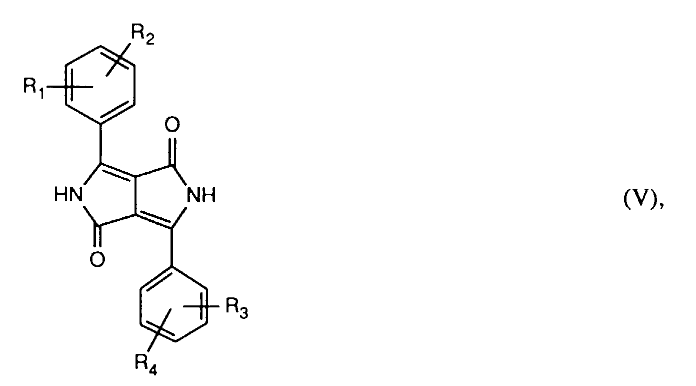 worin R₃ eine Gruppe
-X₂-T-A
bedeutet,
R₁, R₂ und R₄ unabhängig voneinander Wasserstoff, Chlor, Methyl, Methoxy, Cyano oder Phenyl sind und R₁ zusätzlich auch eine Gruppe
-X₂-T-A
bedeuten kann,
X₂ -O- oder -N(R₅)- ist,
T eine Gruppe 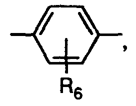 -(CH₂)ₘ- oder eine direkte Bindung bedeutet,
R₅ und R₆ Wasserstoff oder Methyl sind,
A eine Gruppe 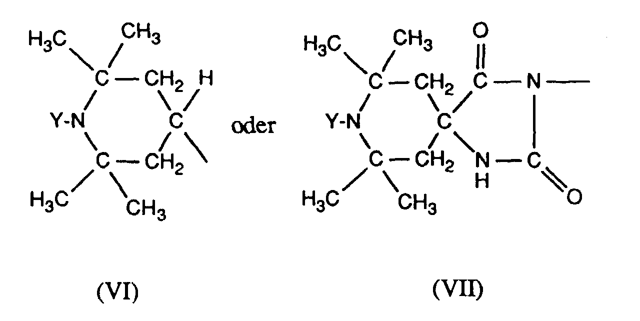 bedeutet, worin
Y O· oder OR ist und R tert.-Butyl, n-Octyl oder Cyclohexyl bedeutet.

4. Diketopyrrolopyrrole gemäss Anspruch 3, der Formel 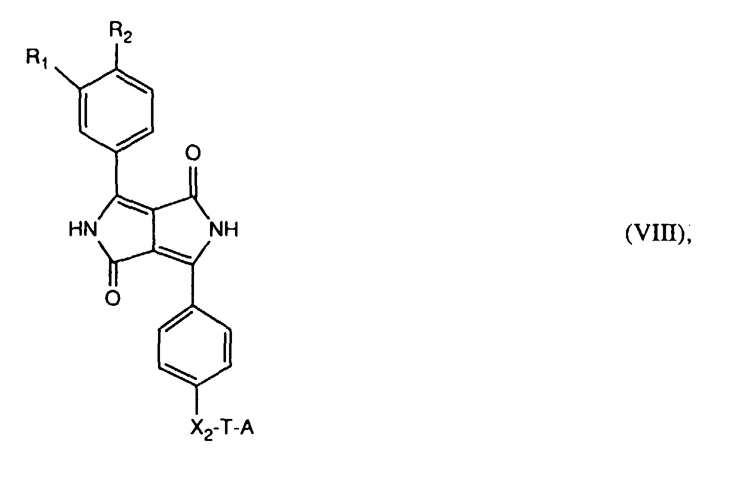 worin einer von R₁ und R₂ Wasserstoff, Chlor, Methyl, Phenyl oder Cyano und der andere Wasserstoff bedeutet,
X₂ -O- bedeutet
T eine Gruppe 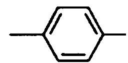 oder eine direkte Bindung ist und
A eine Gruppe der Formel VI bedeutet, worin Y O· ist.

5. Pigmentzusammensetungen enthaltend
a) mindestens ein Diketopyrrolopyrrol der Formel 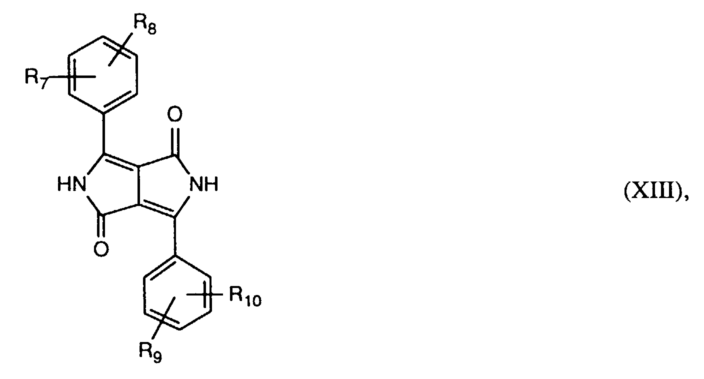 worin R₇, R₈, R₉ und R₁₀ unabhängig voneinander Wasserstoff, Chlor, C₁-C₄-Alkyl, Methoxy, Phenyl oder Cyano bedeuten und
b) 0,1 bis 20 Gew.%, bezogen auf das Diketopyrrolopyrrol a) eines Diketopyrrolopyrrols der Formel I gemäss Anspruch 1.

6. Mit einem Diketopyrrolopyrrol gemäss Anspruch 1 pigmentiertes hochmolekulares organisches Material.

7. Mit einer Pigmentzusammensetzung gemäss Anspruch 5 pigmentiertes hochmolekulares organisches Material.

8. Hochmolekulares organisches Material gemäss Anspruch 6, dadurch gekennzeichnet, dass es sich um einen Lack, insbesondere um einen Automobillack handelt.

9. Hochmolekulares organisches Material gemäss Anspruch 7, dadurch gekennzeichnet, dass es sich um einen Lack, insbesondere um einen Automobillack handelt.

## Claims

1. A diketopyrrolopyrrole of formula 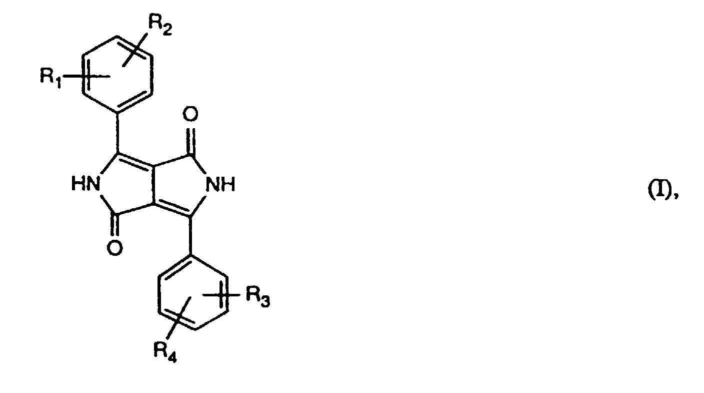 wherein
R₁, R₂, R₃ and R₄ are each independently of one another hydrogen, chloro, C₁-C₄alkyl, methoxy, phenyl, cyano or a group
-X₁-V-X₂-T-A,
where at least one of R₁, R₂, R₃ or R₄ contains the group A,
X₁ and X₂ are each independently of the other -O-, -S-, -N(R₅)-, -CO- or -SO₂- or a direct bond,
V is a group -(CH₂)ₘ-, 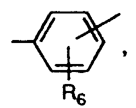 -(CH₂CH₂O)ₘ-CH₂CH₂- or a direct bond and m is 1 or 2, with the proviso that, if V is a direct bond, X₁ is also a direct bond,
T is a group 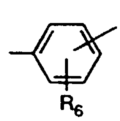 or -(CH₂)ₘ- and, if not linked to a nitrogen atom,
may also be a direct bond,
R₅ is hydrogen or C₁-C₄alkyl and
R₆ is hydrogen, halogen or C₁-C₄alkyl,
A is a group of formula 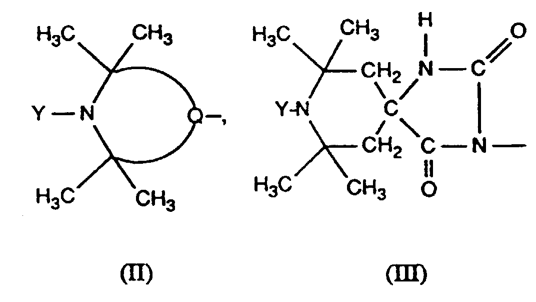 or 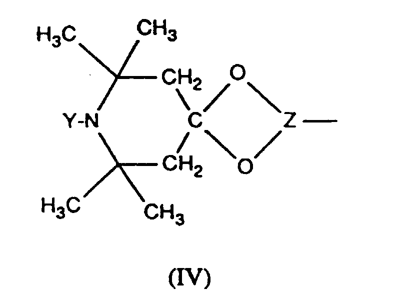 wherein
Y is O·, OH or OR, and R is C₁-C₁₂alkyl or C₅-C₁₂cycloalkyl,
Q is a group of formula 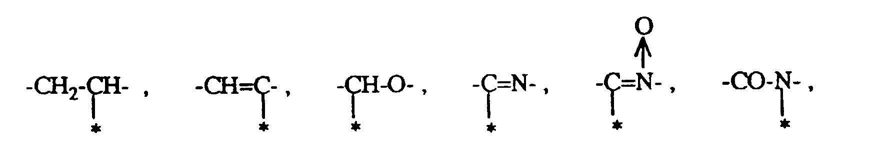 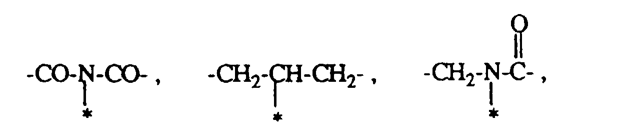 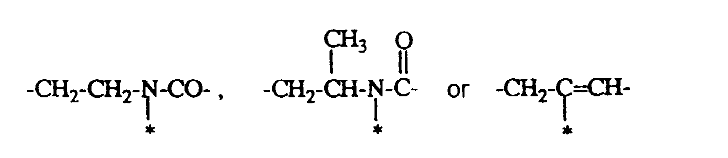 wherein the starred bond is the bond leading to T, and
Z is a group of formula 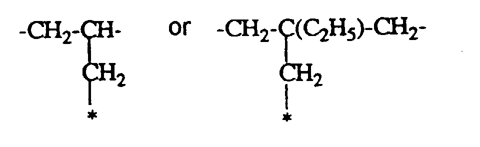 wherein the starred bond is the bond leading to T.

2. A diketopyrrolopyrrole according to claim 1 of formula I, wherein Y is O· or OR, and R is tert-butyl, C₇-C₁₂alkyl or C₅-C₆cycloalkyl.

3. A diketopyrrolopyrrole according to claim 2 of formula 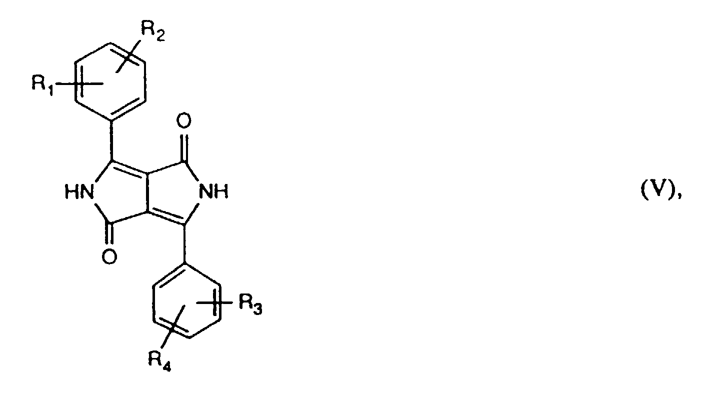 wherein R₃ is a group
-X₂-T-A,
R₁, R₂ and R₄ are each independently of one another hydrogen, chloro, methyl, methoxy, cyano or phenyl, and R₁ may additionally be a group
-X₂-T-A,
X₂ is -O- or -N(R₅)-,
T is a group 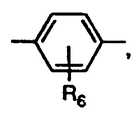 -(CH₂)ₘ- or a direct bond,
R₅ and R₆ are hydrogen or methyl,
A is a group 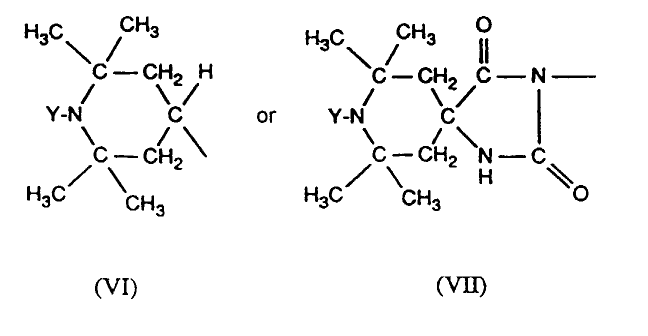 wherein
Y is O· or OR, and R is tert-butyl, n-octyl or cyclohexyl.

4. A diketopyrrolopyrrole according to claim 3 of formula 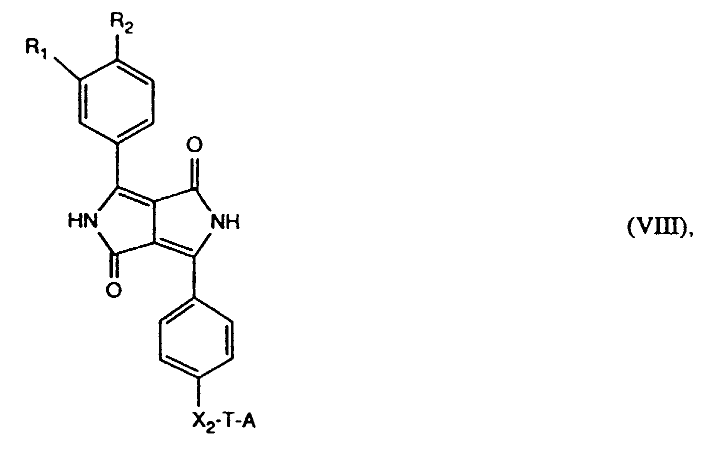 wherein one of R₁ and R₂ is hydrogen, chloro, methyl, phenyl or cyano and the other is hydrogen,
X₂ is -O-,
T is a group 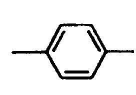 or a direct bond, and
A is a group of formula VI wherein Y is O·.

5. A pigment composition comprising
a) at least one diketopyrrolopyrrole of formula 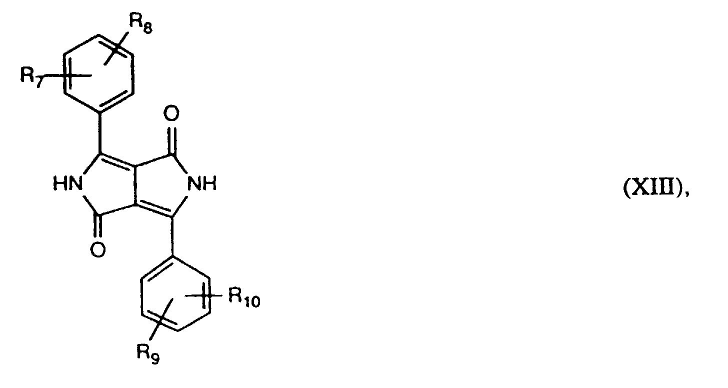 wherein R₇, R₈, R₉ and R₁₀ are each independently of the other hydrogen, chloro, C₁-C₄alkyl, methoxy, phenyl or cyano, and
b) 0.1 to 20 % by weight, based on the diketopyrrolopyrrole a), of a diketopyrrolopyrrole of formula I according to claim 1.

6. High molecular weight organic material pigmented with a diketopyrrolopyrrole according to claim 1.

7. High molecular weight organic material pigmented with a pigment composition according to claim 5.

8. High molecular weight organic material according to claim 6, which is a paint system, especially an automotive finish.

9. High molecular weight organic material according to claim 7, which is a paint system, especially an automotive finish.

## Revendications

1. Dicétopyrrolopyrroles de formule 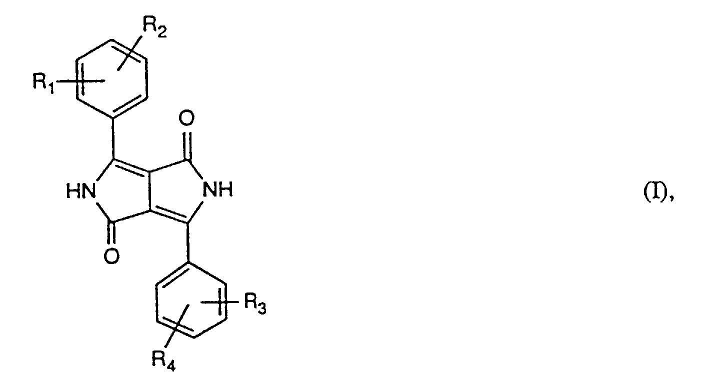 dans laquelle
R₁, R₂, R₃ et R₄, indépendamment les uns des autres, représentant chacun un atome d'hydrogène ou un groupe chloro, alkyle en C₁-C₄, méthoxy, phényle, cyano ou un groupe
-X₁-V-X₂-T-A
où au moins l'un des radicaux R₁, R₂, R₃ ou R₄ contient le groupe A,
X₁ et X₂, indépendamment l'un de l'autre, représentent chacun -O-, -S-, -N(R₅)-, -CO- ou -SO₂-, ou une liaison directe
V est un groupe
-(CH₂)ₘ-, 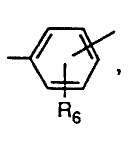 -(CH₂CH₂O)ₘ-CH₂CH₂- ou une liaison directe, et m vaut 1 ou 2, auquel cas, quand V est une liaison directe, X₁ est lui aussi une liaison directe,
T est un groupe 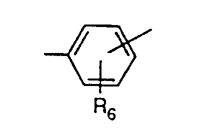 ou -(CH₂)ₘ-, et quand il n'est pas lié à un atome d'azote, peut aussi représenter une liaison directe,
R₅ est un atome d'hydrogène ou un groupe alkyle en C₁-C₄, et
R₆ est un atome d'hydrogène ou un groupe halogéno ou alkyle en C₁-C₄,
A est un groupe de formule 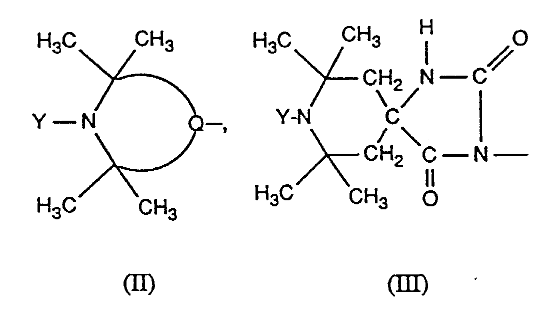 ou 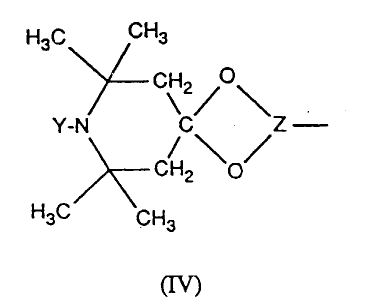 où
Y est O·, OH ou OR, et R est un groupe alkyle en C₁-C₁₂ ou cycloalkyle en C₅-C₁₂,
Q est un groupe de formule 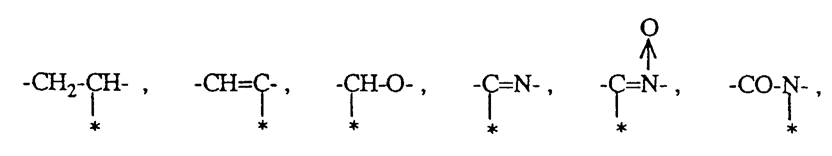 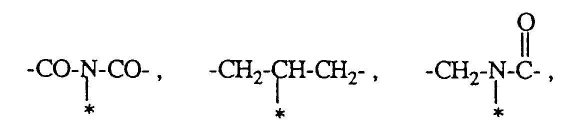 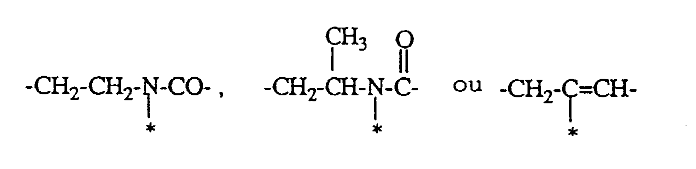 où la liaison désignée par une * représente la liaison conduisant à T, et Z est un groupe de formule 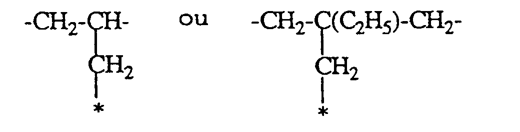 où la liaison désignée par une * représente la liaison conduisant à T.

2. Dicétopyrrolopyrroles selon la revendication 1 de formule I, dans laquelle Y est O· ou OR et R est un groupe tert-butyle, alkyle en C₇-C₁₂ ou cycloalkyle en C₅-C₆.

3. Dicétopyrrolopyrroles selon la revendication 2, de formule 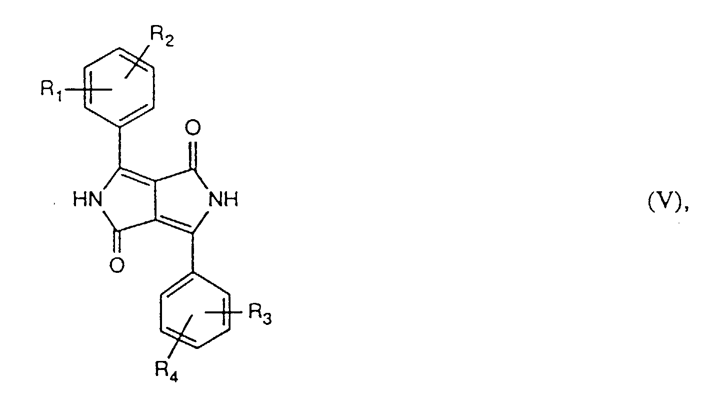 dans laquelle R₃ est un groupe -X₂-T-A,
R₁, R₂ et R₄, indépendamment les uns des autres, représentent chacun un atome d'hydrogène ou un groupe chloro, méthyle, méthoxy, cyano ou phényle, et R₁ peut aussi représenter en outre un groupe -X₂-T-A,
X₂ est -O- ou -N(R₅)-,
T est un groupe 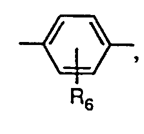 -(CH₂)ₘ-
ou une liaison directe,
R₅ et R₆ représentent chacun un atome d'hydrogène ou le groupe méthyle,
A est un groupe 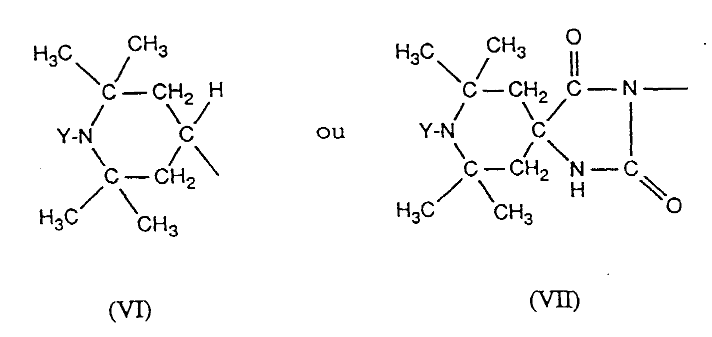 où Y est O· ou OR, et R est un groupe tert-butyle, n-octyle ou cyclohexyle.

4. Dicétopyrrolopyrroles selon la revendication 3, de formule 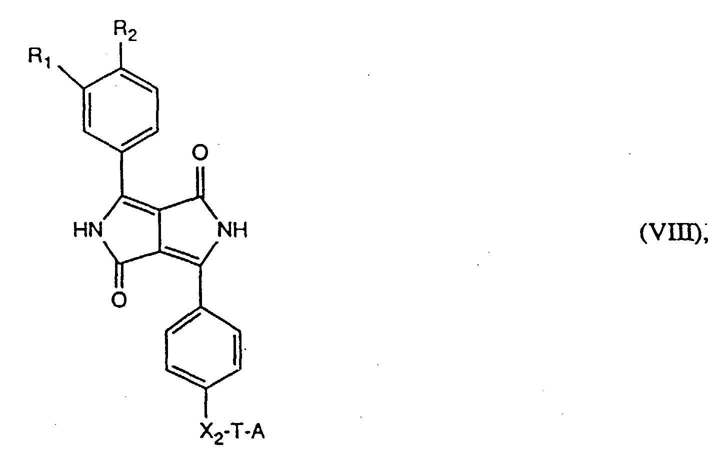 dans laquelle l'un des radicaux R₁ et R₂ est un atome d'hydrogène ou un groupe chloro, méthyle, phényle ou cyano et l'autre est un atome d'hydrogène,
X₂ est -O-,
T est un groupe 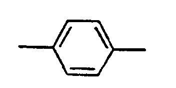 ou une liaison directe, et
A est un groupe de formule VI dans laquelle Y est O·.

5. Compositions pigmentaires contenant
a) au moins un dicétopyrrolopyrrole de formule 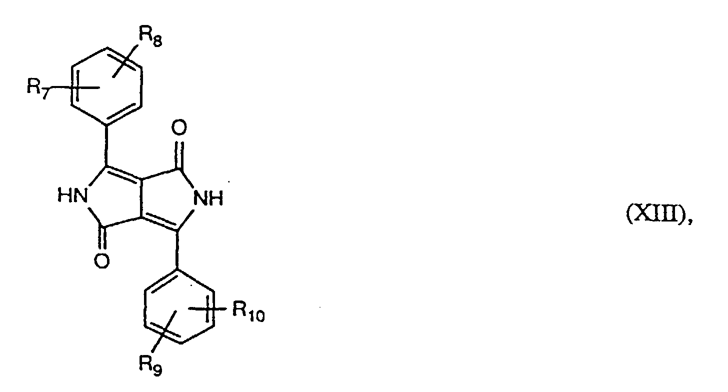 dans laquelle R₇, R₈, R₉ et R₁₀ sont chacun indépendamment des autres un atome d'hydrogène ou un groupe chloro, alkyle en C₁-C₄, méthoxy, phényle ou cyano, et
b) de 0,1 à 20 % en poids, par rapport au dicétopyrrolopyrrole a), d'un dicétopyrrolopyrrole de formule I selon la revendication 1.

6. Matériau organique à grande masse moléculaire, pigmenté par un dicétopyrrolopyrrole selon la revendication 1.

7. Matériau organique à grande masse moléculaire, pigmenté par une composition pigmentaire selon la revendication 5.

8. Matériau organique à grande masse moléculaire selon la revendication 6, caractérisé en ce qu'il s'agit d'un vernis, en particulier pour carrosserie automobile.

9. Matériau organique à grande masse moléculaire selon la revendication 7, caractérisé en ce qu'il s'agit d'un vernis, en particulier d'un vernis pour carrosserie automobile.
